# EUROPEAN PATENT APPLICATION

(11) **EP 4 215 178 A1**
(43) Date of publication of application: **26.07.2023**
(21) Application number: 21865410.1
(22) Date of filing: 13.09.2021
(51) Int. Cl.: A61K 8/64, A61Q 19/00, A61K 38/06

(54) **COMPOSITION, USE OF THE COMPOSITION, COSMETIC METHOD AND METHOD FOR MODULATING THE PRODUCTION OF SEBUM**

(30) Priority: 11.09.2020 BR 102020018531
(71) Applicant: Chemyunion Ltda, 18087-101 Sorocaba, SP (BR)
(72) Inventor: KATEKAWA, Edson, 18087-180 Sorocaba/SP (BR); GUIDO, Rafael Victorio Carvalho, 13565-090 Sorocaba/SP (BR); NOGUEIRA, Cecília, 18085-844 Sorocaba/SP (BR); MUSSI, Lilian, 18013-240 Sorocaba/SP (BR); JUNIOR, Flávio Bueno De Camargo, 18013-004 Sorocaba/SP (BR); MAGALHÃES, Wagner Vidal, 18090-360 Sorocaba/SP (BR); PRINCIVAL, Cleverson Rogério, Jardim Moncayo Sorocaba (BR)
(74) Representative: Pace Napoleone, Maria
(86) International application number: PCT/BR2021/050392
(87) International publication number: WO 2022/051831

(57) **Abstract**

The present invention describes the use of peptide compounds to reduce the production of skin sebum. In one embodiment, the present invention discloses a composition comprising peptide compounds and their use for cosmetic treatments or skin diseases and disorders, such as acne vulgaris. The present invention refers to the fields of chemistry, pharmacy, and cosmetology.

## Description

### Field of the Invention

The present invention describes the use of peptide compounds to reduce the production of skin sebum. In one embodiment, the present invention discloses a composition comprising peptide compounds and their use for cosmetic treatments or skin diseases and disorders, such as acne vulgaris. The present invention refers to the fields of chemistry, pharmacy, and cosmetology.

### Background of the Invention

The reduction of skin oiliness can be approached in several ways, with the most accessible being the frequent removal of the sebum produced that reaches the skin surface. This can be achieved by using cleansing agents, such as soaps, detergents, and solvents, possibly with the aid of pore cleansing agents that facilitate the release of the sebum produced by the sebaceous glands. However, in case of sebum overproduction, this may not be a feasible or effective solution; in such cases, the reduction of sebum production is necessary. The treatments available on the market for this approach are based on retinoids or 5-alpha reductase enzyme inhibitors.

Isotretinoin is a highly potent sebum-production inhibitor retinoid. It is believed that this effect is related to the induction of apoptosis in several types of cells, including sebocytes in the sebaceous gland (NELSON et al., 2006). However, the list of adverse effects is wide and well known, and therefore its use in fighting oily skin can be considered controversial. Other retinoids, such as tretinoin and adapalene, remain as options, but may cause skin irritation characterized by redness and desquamation; in addition, their use require medical guidance (CHIEN, 2018). Examples of patent documents describing the use of retinoids to reduce the production of sebum in human skin are: EP0552624 A1, US2015209343 A1.

It is known that sexual hormones act positively in the production of sebum (SWEENEY et al., 1969). Dihydrotestosterone (DHT) is the most potent known male hormone and is produced by the body through the action of 5-alpha reductase (5AR) enzyme on testosterone. Thus, 5AR was established as one of the main targets against excessive skin oiliness. Among the cosmetic actives that use this mechanism of action, one can mention Sepicontrol A5 (SEPPIC), Zincidone (SOLABIA), Cytobiol Iris A2 (GATTEFOSSÉ), Panadoxine P (DAIICHI FINE CHEMICALS), Teavigo (DSM) and 5 Alpha Avocuta Skin (LABORATOIRES EXPANSCIENCE). However, clinical and *in vitro* studies indicate that the isolated suppression of 5AR is not sufficient to control oiliness in cosmetic or therapeutic applications (LEYDEN et al., 2004; SEIFFERT et al., 2007). In addition, side effects have already been reported on the use of 5AR inhibitors, so that such a strategy may not be considered sufficiently safe (FERTIG et al., 2016). Examples of patent documents describing the use of 5-alpha reductase inhibitors aiming to reduce the production of sebum in human skin are: WO9637201 A2, FR2765105 A1 and FR2731352 A1.

It has been recently reported that olumacostat glasaretil (OG), an acetyl-CoA carboxylase (ACC) enzyme inhibitor, could reduce sebum production (HUNT et al., 2017). The clinical studies published so far, however, only report the number of acne lesions and the reduction in sebum production was not directly evaluated. Patent US4529587A mentions the use of the same mechanism (ACC antagonism) to reduce sebum production.

Other substances, such as green tea extract (MAHMOOD et al., 2010), lotus extract (MAHMOOD et al., 2013), niacinamide (DRAELOS et al., 2006) and carnitine (PEIRANO et al., 2012), have also been reported as agents reducing sebum production, although with limited commercial efficacy or acceptance.

In the search for the state of the art in scientific and patent literature, the following documents dealing with the subject were found:

US2018305419A1 describes the use of polypeptides with at least six amino acids to reduce sebum production.

CN107325157 describes the use of polypeptides with at least nine amino acids to reduce sebum production.

Thus, from what is evident from the literature researched, no documents were found that anticipate or suggest the teachings of the present invention, so that the solution proposed herein has novelty and inventive activity compared to the prior art.

In the prior art, there remains a need for simpler, and more efficient compounds to control the production of skin sebum without side effects from such compounds.

### Summary of the Invention

Thus, the present invention solves the problems of the prior art by using peptide compounds for modulating the production of skin sebum and its use in pharmaceutical and/or cosmetic compositions.

In a first object, the present invention relates to a composition for modulating the production of sebum in the skin of a mammal comprising from 0.001 % (w/w) to 10.00% (w/w) of at least one compound of the general formula

(I) R1-AA1-AA2-AA3-R2,

wherein
AA1 is an amino acid containing polar side chain with a negative (partial or formal) charge and selected from the group consisting of Asp, Glu, Ser, and Thr;
AA2 is an amino acid containing nonpolar side chain and selected from the group consisting of Ala, Ile, Leu, and Val;
AA3 is an amino acid containing aromatic side chain and selected from the group consisting of Phe, Trp, and TyrR1 is a N-terminal chain modifier selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls;
R2 is selected from the group consisting of -NR3R4, OR3, and -SR3, wherein R3 and R4 are independently selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls.

In a second object, the present invention relates to the use of a composition to prepare a composition for dermatological or cosmetic treatment, wherein the composition comprises from 0.001% (w/w) to 10.00% (w/w) of at least one compound of the general formula (I) R1-AA1-AA2-AA3-R2 wherein,
AA1 is an amino acid containing polar side chain with a negative (partial or formal) charge and selected from the group consisting of Asp, Glu, Ser, and Thr;
AA2 is an amino acid containing nonpolar side chain and selected from the group consisting of Ala, Ile, Leu, and Val;
AA3 is an amino acid containing aromatic side chain and selected from the group consisting of Phe, Trp, and Tyr;
R1 is a N-terminal chain modifier selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls;
R2 is selected from the group consisting of -NR3R4, OR3, and -SR3, wherein R3 and R4 are independently selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls.

In a third object, the present invention relates to a method for modulating the production of sebum in the skin of a mammal, comprising the application of a composition comprising from 0.001% (w/w) to 10.00% (w/w) of at least one compound of the general formula (I) R1-AA1-AA2-AA3-R2 wherein,
AA1 is an amino acid containing polar side chain with a negative (partial or formal) charge and selected from the group consisting of Asp, Glu, Ser, and Thr;
AA2 is an amino acid containing nonpolar side chain and selected from the group consisting of Ala, Ile, Leu, and Val;
AA3 is an amino acid containing aromatic side chain and selected from the group consisting of Phe, Trp, and Tyr;
R1 is a N-terminal chain modifier selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls;
R2 is selected from the group consisting of -NR3R4, OR3, and -SR3, wherein R3 and R4 are independently selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls.

In a fourth object, the present invention relates to a cosmetic method for decreasing the oiliness of the skin of a mammal, comprising the application of a cosmetic composition comprising at least one pharmaceutically acceptable excipient and from 0.001% (w/w) to 10.00% (w/w) of at least one compound of the general formula (I) R1-AA1-AA2-AA3-R2 wherein,
AA1 is an amino acid containing polar side chain with a negative (partial or formal) charge and selected from the group consisting of Asp, Glu, Ser, and Thr;
AA2 is an amino acid containing nonpolar side chain and selected from the group consisting of Ala, Ile, Leu, and Val;
AA3 is an amino acid containing aromatic side chain and selected from the group consisting of Phe, Trp, and Tyr;
R1 is a N-terminal chain modifier selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls;
R2 is selected from the group consisting of -NR3R4, OR3, and -SR3, wherein R3 and R4 are independently selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls.

These and other objects of the invention will be immediately appreciated by those skilled in art and will be described in detail below.

### Brief Description of Figures

The following figures are shown:
Fig. 1 shows the effect of SEQ ID No. 1 on sebogenesis in SEBO662AR sebocyte models, containing androgen-free lipogenic medium.
Fig. 2 shows a bar graph with the mean of the sebum-metric indices before (D0) and after 14 (D14) days of continuous use in the placebo group.
Fig. 3 shows a bar graph with the mean of the sebum-metric indices before (D0) and after 28 (D28) days of continuous use in the placebo group.
Fig. 4 shows a bar graph with the mean of the sebum-metric indices before (D0) and after 14 (D14) days of continuous use in the group that used the product (BC-tripeptide) twice a day (morning and evening).
Fig. 5 shows a bar graph with the mean of the sebum-metric indices before (D0) and after 28 (D28) days of continuous use in the group that used the product (BC-tripeptide) twice a day (morning and evening).

### Detailed Description of the Invention

Sebum is a substance produced in the sebaceous glands and secreted into the skin through a holocrine process. Composed of the whole contents of the sebocytes cytoplasm, it is a material rich in lipids and has the function of lubricating and waterproofing the skin. Uncontrolled sebum production, notably the overproduction, contributes to several skin conditions, including one of the most prevalent worldwide: acne vulgaris.

The present invention relates to the use of tripeptide-based compounds for modulating the production of skin sebum or the production of lipids in adipose tissue.

The peptides showed in the present invention are safe and effective substances for reducing the production of sebum, with evidence through *in vitro* tests, in which no cytotoxic potential for these peptides was reported. Additionally, they are peptides composed of a smaller number of amino acids than those already reported for this type of application.

Such tripeptides have the general formula (I) R1-AA1-AA2-AA3-R2, wherein:
AA1 is an amino acid containing polar side chain with a negative (partial or formal) charge and selected from the group -Asp-, -Glu-, -Ser- and -Thr;
AA2 is an amino acid containing nonpolar side chain and selected from the group -Ala-, -Ile-, Leu- and -Val-; AA3 is an amino acid containing aromatic side chain and selected from the group -Phe-, -Trp- and -Tyr;
R1 is a N-terminal chain modifier selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls;
R2 is selected from the group consisting of -NR3R4, OR3, and -SR3, wherein R3 and R4 are independently selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls.

The present invention includes any and all conservative substitution of SEQ ID No. sequence 1. Conservative substitution means the substitution of one amino acid by another with similar hydrophobicity, polarity, and/or side chain, so that there is little or no three-dimensional or functional change of the peptide. Furthermore, it is understood in the present invention that the term "compound" includes its hydrates, stereoisomers, multimers, cyclic forms, drug-conjugates, prodrugs and cosmetically or pharmaceutically acceptable salts thereof.

Among these peptides there are, as examples, H-Glu-Val-Phe-OH (SEQ ID No. 1) and Palm-Glu-Val-Phe-OH (or Palm-SEQ ID No. 1), H-Asp-Ala-Phe-OH (SEQ ID No. 2) and Palm-Asp-Ala-Phe-OH (or Palm-SEQ ID No. 2), H-Ser-Leu-Trp-OH (SEQ ID No. 3) and Palm-Ser-Leu-Trp-OH (or Palm-SEQ ID No. 3), H-Thr-Ile-Tyr-OH (SEQ ID No. 4) and Palm-Thr-Ile-Tyr-OH (or Palm-SEQ ID No. 4), with the Palm- prefix identifying an N-palmitoylated peptide.

Such peptides can be obtained through several methods, preferably by chemical synthesis, without excluding other possibilities, such as biotechnology, hydrolysis and/or purification of proteins and other methods known in the prior art.

These peptides can be used in a concentration range from 0.001% (w/w) to 10.00% (w/w), preferably about 0.01% (w/w), applied in a cosmetic or pharmaceutical formulation suitable for human or veterinary use.

A cosmetic or pharmaceutical formulation suitable for human or veterinary use means all and any type of solvent, dispersion medium, encapsulation, permeation enhancers, surfactants, preservatives, and other ingredients, both alone and in combination, and which are physiologically compatible.

In a first object, the present invention relates to a composition for modulating the production of sebum in the skin of a mammal comprising from 0.001% (w/w) to 10.00% (w/w) of at least one compound of the general formula

(I) R1-AA1-AA2-AA3-R2,

wherein
AA1 is an amino acid containing polar side chain with a negative (partial or formal) charge and selected from the group consisting of Asp, Glu, Ser, and Thr;
AA2 is an amino acid containing nonpolar side chain and selected from the group consisting of Ala, Ile, Leu, and Val;
AA3 is an amino acid containing aromatic side chain and selected from the group consisting of Phe, Trp, and TyrR1 is a N-terminal chain modifier selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls;
R2 is selected from the group consisting of -NR3R4, OR3, and -SR3, wherein R3 and R4 are independently selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls.

In one embodiment, R1 and R2 are not proteinogenic amino acids.

It is understood in the present invention that amino acids may contain acceptable chemical modifications in the side chains. Non-limiting examples of acceptable chemical modifications include acetylation, acylation, ADP ribosylation, amidation, branching, cross-linking, cyclization, disulfide bond formation, demethylation, glycosylation, hydroxylation, iodination, methylation, oxidation, phosphorylation, prenylation, racemization, selenoylation, sulfation, and ubiquitination, complexation with metal ions.

In one embodiment, R1 and R2 comprise acetyl groups, carbonyl group, methoxy fluorenyl group, formyl group, palmitoyl group, myrisityl group, stearyl or polyethylene glycol (PEG) groups. In one embodiment, the group R1 is palmitoyl.

In one embodiment, the peptide sequence AA1-AA2-AA3 is as defined by SEQ ID No. 1, SEQ ID No. 2, SEQ ID No. 3 or SEQ ID No. 4.

In one embodiment, the composition comprises from 0.005% to 7% of the compound. In one embodiment, the composition comprises from 0.01% to 5% (w/w) of the compound. In one embodiment, the composition comprises 0.01% (w/w) of compound.

In one embodiment, the composition comprises at least one pharmaceutically/cosmetically acceptable excipient selected from the group consisting of solubilizers, preservatives, emulsifiers, carriers, colorants, antioxidant additives, fillers, surfactants, UVA and/or UVB filters, fragrances, thickeners, humectants, whitening agents, antimycotic agents, antiparasitic agents, external analgesics, sunscreens, photoprotectors, keratolytic agents, detergents/surfactants, moisturizers, nutrients, vitamins, energy enhancers, antiperspirants, astringents, deodorants, hair removers, hardening agents, anti-callus agents. In one embodiment, the pharmaceutically/cosmetically acceptable excipient is selected from the group consisting of emulsifier, preservative and solubilizer.

In one embodiment, the composition additionally comprises from 10% to 20% (w/w) of an emulsifier. In one embodiment, the composition comprises from 10% to 15% of an emulsifier. In one embodiment, the emulsifiers are selected from the group consisting of fatty alcohols, ethoxylated fatty alcohols, glyceryl esters and alkyl glycosides.

In one embodiment, the composition additionally comprises from 0.1% to 5% (w/w) of a preservative. In one embodiment, the composition additionally comprises from 0.3% to 2% (w/w) of a preservative. In one embodiment, the preservatives are selected from the group consisting of xylitol esters, benzyl alcohol, benzyl ethers, and potassium sorbate.

In one embodiment, the composition additionally comprises from 0.1% to 5% (w/w) of a solubilizer. In one embodiment, the composition additionally comprises from 0.3% to 2% (w/w) of a solubilizer. In one embodiment, the solubilizer is selected from the group consisting of water, propanediol, glycerol, esters, and ethers.

In one embodiment, the composition may be presented in the form of powders, creams, ointments, lotions, sprays, suspensions, gel, cream-gel, microemulsion, nanoemulsion, soap, hand/bath soap, moisturizers, sunscreens, mask, oil, wipes, balms. In one embodiment, the compound is encapsulated; non-exhaustive examples include microencapsulation and nanoencapsulation.

In one embodiment, the composition comprises additional actives, such as other anti-acne/sebum production controlling actives, anti-inflammatory, antiallergic, healing, emollients, humectants, etc. Examples of additional actives comprise corticosteroids, azathioprin, bisabolol, cyclosporin A, acetylsalicylic acid, ibuprofen, panthenol, chamomile extract or aloe extract, antiphlogistic, cytostatics, alcohols, p-hydrobenzoic esters, imidazolidinyl urea, formaldehyde, sorbic acid, benzoic acid, salicylic acid, zinc ricinoleate, triclosan, undecyleneic acid alkylamides, triethyl citrate, chlorhexidine, azoles, zinc pyritone, selenium sulfides, azelaic acid, potassium azelaoyl diglycinate, sebacic acid, 10-hydroxydecanoic acid, 1,10-decanediol, aluminum salts.

In a second object, the present invention relates to the use of a composition to prepare a composition for dermatological or cosmetic treatment, wherein the composition comprises from 0.001% (w/w) to 10.00% (w/w) of at least one compound of the general formula (I) R1-AA1-AA2-AA3-R2 wherein,
AA1 is an amino acid containing polar side chain with a negative (partial or formal) charge and selected from the group consisting of Asp, Glu, Ser, and Thr;
AA2 is an amino acid containing nonpolar side chain and selected from the group consisting of Ala, Ile, Leu, and Val;
AA3 is an amino acid containing aromatic side chain and selected from the group consisting of Phe, Trp, and Tyr;
R1 is a N-terminal chain modifier selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls;
R2 is selected from the group consisting of -NR3R4, OR3, and -SR3, wherein R3 and R4 are independently selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls.

In one embodiment, the compound is used to prepare a medicament for the treatment of acne and/or production of skin sebum-associated disorders or diseases. In one embodiment, the compound is used to prepare a composition for the treatment of acne, preferably acne vulgaris. In one embodiment, the compound is used for the treatment of seborrheic dermatitis, rosacea, eczema, psoriasis, actinic keratosis, bedsores, sebaceous cysts, inflammatory dermatoses, post-inflammatory hyperpigmentation, miliaria, hyperlipidemia, hyperandrogenism, seborrhea, sebaceous hyperplasia, follicular rash, oily skin, keratinocyte cyst, hypertrichosis, dandruff, follicular infections, pseudofolliculitis barbae, follicular hyperkeratosis. In one embodiment, the compound is used for the treatment of acne, alopecia, dandruff, seborrheic dermatitis, sebaceous hyperplasia, nevus sebaceous, psoriasis, rosacea, seborrhea.

In a third object, the present invention relates to a method for modulating the production of sebum in the skin of a mammal, comprising the application of a composition comprising from 0.001% (w/w) to 10.00% (w/w) of at least one compound of the general formula (I) R1-AA1-AA2-AA3-R2 wherein,
AA1 is an amino acid containing polar side chain with a negative (partial or formal) charge and selected from the group consisting of Asp, Glu, Ser, and Thr;
AA2 is an amino acid containing nonpolar side chain and selected from the group consisting of Ala, Ile, Leu, and Val;
AA3 is an amino acid containing aromatic side chain and selected from the group consisting of Phe, Trp, and Tyr;
R1 is a N-terminal chain modifier selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls;
R2 is selected from the group consisting of -NR3R4, OR3, and -SR3, wherein R3 and R4 are independently selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls.

In a fourth object, the present invention relates to a cosmetic method for decreasing the oiliness of the skin of a mammal, comprising the application of a cosmetic composition comprising at least one pharmaceutically acceptable excipient and from 0.001% (w/w) to 10.00% (w/w) of at least one compound of the general formula (I) R1-AA1-AA2-AA3-R2 wherein,
AA1 is an amino acid containing polar side chain with a negative (partial or formal) charge and selected from the group consisting of Asp, Glu, Ser, and Thr;
AA2 is an amino acid containing nonpolar side chain and selected from the group consisting of Ala, Ile, Leu, and Val;
AA3 is an amino acid containing aromatic side chain and selected from the group consisting of Phe, Trp, and Tyr;
R1 is a N-terminal chain modifier selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls;
R2 is selected from the group consisting of -NR3R4, OR3, and -SR3, wherein R3 and R4 are independently selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls.

In one embodiment, the composition is applied daily. In one embodiment, the composition is applied 2 to 3 times a day. In one embodiment, the composition is applied topically.

The products can be used as cosmetics, cosmeceuticals, or topical drugs capable of modulating the production of skin sebum. This list of applications includes cosmetics for modulating skin and scalp oiliness, astringent or skin moisturizing lotions, cosmetics for reducing the diameter of the skin pores, anti-acne products, among others.

The present invention presents alternatives for reducing the production of skin sebum through a safe topical treatment, without the interference with sex hormones, or use of retinoids.

### Examples

The examples shown here are intended only to exemplify one of the numerous ways of making the invention, however, without limiting its scope.

### Sebum Production Inhibition

Sebocytes are cells found in the sebaceous glands and that, in the differentiation process, accumulate lipid droplets in their interior. The SEBO662AR cell model is an immortalized lineage of human sebocytes that responds to lipogenic stimuli and that can be used to select potential sebum production inhibitors.

A sebum production inhibition study was conducted in the SEBO662AR cell model cultivated in androgen-free lipogenic medium, and the formation of droplets was monitored by using Bodipy^{®} fluorescent probes and image analysis. As an inhibition reference, cerulenin was applied at a concentration of 10 micromolar - a fatty acid synthase inhibitor which, although effective in reducing sebum production, is a very labile molecule as it has unsaturations and an epoxy group in its structure, so that its use is restricted to laboratory testing. In this study, SEQ ID No. 1 led to a 28% reduction in sebum production when applied at a concentration of 500 micromolar (higher non-cytotoxic concentration), comparable to that observed with cerulenin.

### Pharmaceutical Composition

Additionally, the compounds of the present invention can be used in several pharmaceutical compositions (Table 1). These compositions also led to a reduction in sebum production.

**Table 1. Pharmaceutical composition**

| **Phase** | **INCI** | **% (w/w)** | **Function** |
|---|---|---|---|
| A | Cetearyl alcohol and polysorbate 60 | 12.00 | Emulsifier |
| B | Aqua | 100.00 | Solvent |
| C | Phenoxyethanol | 0.50 | Preservative |
| | Propanediol | 1.00 | Solubilizer |
| | Tripeptide (Glu-Val-Phe) | 0.01 | Active |

### Clinical Testing

For the clinical efficacy evaluation, the tripeptide (Glu-Val-Phe) was applied in a cosmetic base (BC) at a concentration of 0.01% (w/w) and compared to a placebo base, without active (Table 2).

**Table 2. Formulation of the analyzed samples:**

| Cosmetic Base Components | Components %(w/w) | | Function |
|---|---|---|---|
| | BC-Placebo | BC-tripeptide | |
| Water | q.s. 100.00 | q.s. 100.00 | Solvent |
| Cetostearyl alcohol (and) polysorbate 60 | 12 | 12 | Emulsifier |
| Tripeptide | - | 0.01 | Active |

The study was conducted with 20 female volunteers, between 18 and 50 years old, with mixed or oily skin, oily skin with a sebum-metric index above 100, who used the product (BC-placebo or BC-tripeptide) twice a day (morning and evening) for 28 days.

### Intermediate Visit (V02-D14 ± 2 days).

The participants were referred to the Instrumental Sector for acclimatizing for at least 20 minutes, in a room with controlled temperature and humidity (T = 20 ± 2°C and RH = 50 ± 5%). After the acclimatizing period, sebum-metrics measurements were performed on the forehead.

### Final Visit (V03-D28 ± 2 days).

The participants were referred to the Instrumental Sector for acclimatizing for at least 20 minutes, in a room with controlled temperature and humidity (T = 20 ± 2°C and RH = 50 ± 5%). After the acclimatizing period, sebum-metrics measurements were performed on the forehead. At the end of all procedures, the participants were dismissed, and the study was ended.

### Sebumeter^{®} SM 815 Equipment (Courage & Khazaka) - Skin Oiliness

The Sebumeter photometrically measures the increase in transparency of a special plastic strip that becomes translucent when coated with sebum. The plastic strip is approximately 0.1 mm thick and 64 mm² in area. The strip is supported by a mirror which presses it against the skin of the region to be evaluated with a fixed pressure of 10N by means of a spring. The instrument has a timing device that allows 30-second measurements. The strip transparency is evaluated by a photocell present in the device where the plastic strip is inserted, and the light transmission represents the amount of sebum per area and the results are expressed in absolute values (µg sebum/cm²). The product was applied on the right hemiface and, therefore, the side opposite to the application was considered the control area. Measurements were made in triplicate.

### Results - Reduction of face oiliness

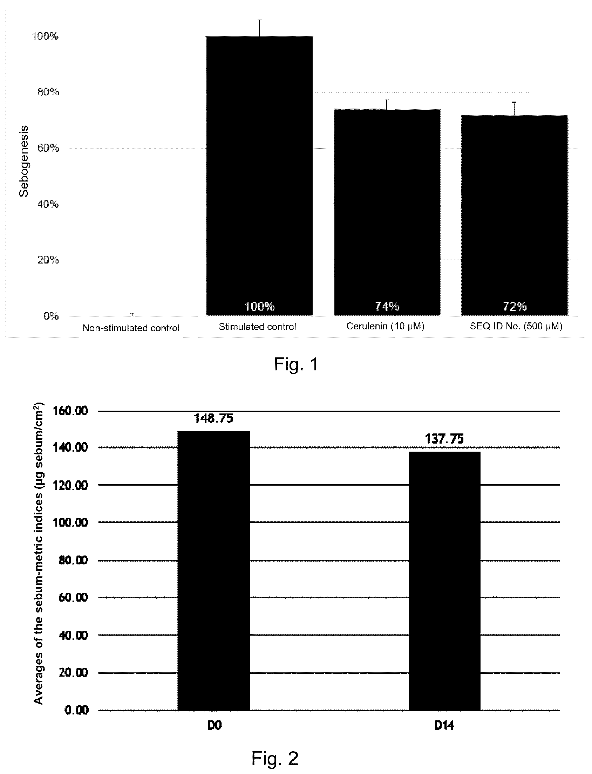

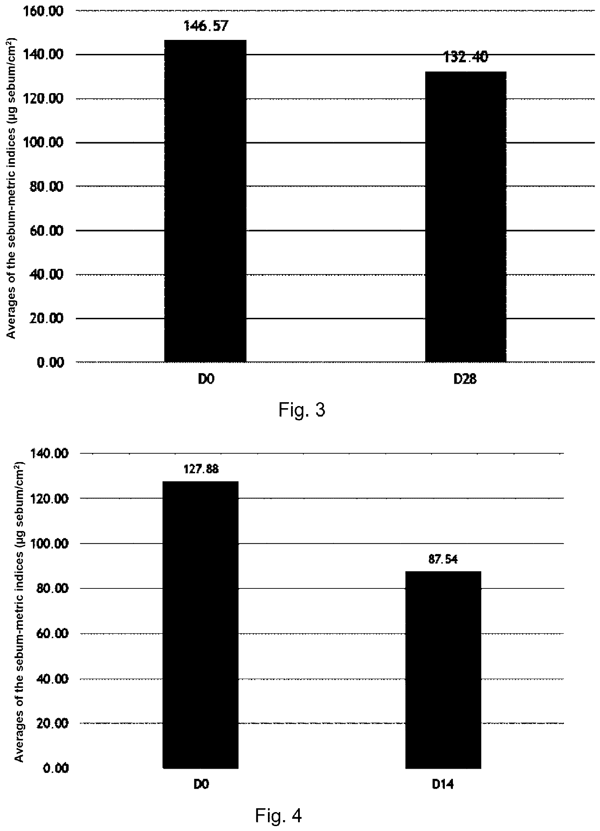

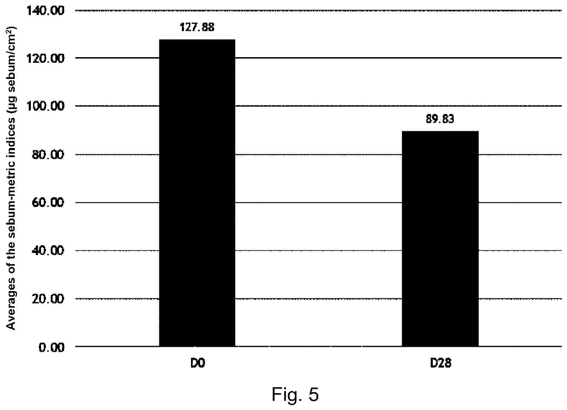

The efficacy of the tripeptide in controlling face oiliness compared to that of BC-Placebo was evaluated by basal measurements of sebum-metrics (sebum-metric index) in triplicate on the participants hemifaces before (D0) and after 14 (D14) (Figs. 2 and 3) and 28 (D28) days (Figs. 4 and 5) of continuous use of the formulations, using a Sebumeter^{®} SM 815 equipment (Courage & Khazaka).

The use of the tripeptide (0.01% w/w) led to a reduction in skin oiliness when compared to the initial time (D0) in the evaluated times. After 14 days of use, the average of the sebum-metric indices decreased by 31.54%. After 28 days of treatment, the use of the tripeptide reduced an average of 29.75% oiliness among the female volunteers. The use of the base without active (BC-placebo), reduced by only 7.39% and 9.67% the average of the sebum-metric indices of the volunteers after 14 and 28 days, respectively. The statistical test was based on the *Anderson-Darling* normality test.

Those skilled in the art will value the knowledge presented herein and may reproduce the invention in the embodiments presented and in other variants and alternatives, covered by the scope of the following claims.

## Claims

1. A composition for modulating the production of sebum in the skin of a mammal, **characterized in that** it comprises from 0.001% (w/w) to 10.00% (w/w) of at least one compound of the general formula (I) R1-AA1-AA2-AA3-R2 wherein,
AA1 is an amino acid containing polar side chain with a negative (partial or formal) charge and selected from the group consisting of Asp, Glu, Ser, and Thr;
AA2 is an amino acid containing nonpolar side chain and selected from the group consisting of Ala, Ile, Leu, and Val;
AA3 is an amino acid containing aromatic side chain and selected from the group consisting of Phe, Trp, and Tyr;
R1 is a N-terminal chain modifier selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls;
R2 is selected from the group consisting of -NR3R4, OR3, and -SR3, wherein R3 and R4 are independently selected from the group consisting of H, substituted or unsubstituted acyclic aliphatics, substituted or unsubstituted cyclic aliphatics, substituted or unsubstituted heterocyclics, substituted or unsubstituted heteroarylalkyl, substituted or unsubstituted aryl, substituted or unsubstituted arylalkyl, substituted or unsubstituted acyl, substituted or unsubstituted carbonyls.

2. The composition according to claim 1, **characterized in that** it reduces the production of sebum.

3. The composition according to claim 1 or 2, **characterized in that** the peptide sequence AA1-AA2-AA3 is as defined by SEQ ID No. 1, SEQ ID No. 2, SEQ ID No. 3 or SEQ ID No. 4.

4. The composition according to any one of claims 1 to 3, **characterized in that** it comprises from 80% to 100% (w/w) of solvent.

5. The composition according to any one of claims 1 to 4, **characterized in that** it comprises at least one pharmaceutically or cosmetically acceptable excipient selected from the group consisting of emulsifier, preservative and solubilizer.

6. The composition according to any one of claims 1 to 5, **characterized in that** it additionally comprises:
- from 10% to 20% (w/w) of an emulsifier;
- from 0.1% to 5% (w/w) of a preservative; and
- from 0.1% to 5% (w/w) of a solubilizer.

7. Use of a composition as defined in any one of claims 1 to 6, **characterized in that** it is to prepare a composition for dermatological or cosmetic treatment.

8. Use of a composition according to claim 7, **characterized in that** it is to prepare a medicament for production of skin sebum-associated disorders or diseases.

9. A method for modulating the production of sebum in the skin of a mammal, **characterized in that** it comprises the application of a composition as defined in any one of claims 1 to 6.

10. A cosmetic method for decreasing the oiliness of the skin of a mammal, **characterized in that** it comprises the application of a composition as defined in any one of claims 1 to 6.
